(19) Europäisches Patentamt
European Patent Office
Office européen des brevets

(11) Veröffentlichungsnummer: **0 004 522 B1**

# (12) EUROPÄISCHE PATENTSCHRIFT

(45) Veröffentlichungstag der Patentschrift: **23.06.82**

(51) Int. Cl.³: **C 07 D 307/80**, C 07 D 307/81, C 07 D 307/86, A 61 K 31/34 // C07C43/205, C07F9/12

(21) Anmeldenummer: **79810025.1**

(22) Anmeldetag: **12.03.79**

(54) **Neue diligno1ähnliche Verbindungen, dilignolähnliche Verbindungen zur Verwendung bei der Behandlung von Lebererkrankungen sowie diese enthaltende pharmazeutische Präparate.**

(30) Priorität: **22.03.78 DE 2812664**

(43) Veröffentlichungstag der Anmeldung: **03.10.79 Patentblatt 79/20**

(45) Bekanntmachung des Hinweises auf die Patenterteilung: **23.06.82 Patentblatt 82/25**

(84) Benannte Vertragsstaaten: **BE CH DE FR GB IT LU NL SE**

(56) Entgegenhaltungen:
**FR-M 7067**
**CHEMICAL ABSTRACTS, Vol. 84, Nr. 13, 29. März 1976, Columbus, Ohio, USA,**
**K. S. AKHMED KHODZHAEVA et al.: »Some pharmacological properties of eudesmin«, Seite 95, Zusammenfassung Nr. 84664q**
**THE JOURNAL OF ANTIBIOTICS, Vol. 29, Nr. 9, September 1976, Tokyo, Y. KUMADA et al.: »Dehydrodicaffeic acid dilactone, an inhibitor of catechol-o-methyl transferase«, Seiten 882–889**
**THE JOURNAL OF ANTIBIOTICS, Vol. 31, Nr. 2, Februar 1978, Tokyo Y. KUMADA et al.: »Biochemical activities of the derivatives of dehydrodicaffeic acid dilactone«, Seiten 105–111**
**DIE NATURWISSENSCHAFTEN, Vol. 44, Nr. 13, Juli 1957, Berlin DE, A. S. RAMASWAMY et al.: »Antitubercular activity of sesamin and other related compounds«, Seite 380**
**K. V. SARKANEN: LIGNINS, OCCURENCE, FORMATION STRUCTURE AND REACTIONS 1971 Wiley-Interscience, New York, USA**

(73) Patentinhaber: **PHARMACEUTICAL LICENCES COMPANY LTD., 7, Chemin de Trembley, CH-1197 Prangins VD (CH)**

(72) Erfinder: **Griengl, Herfried, Argenot-Strasse 23, A-8047 Graz (AT)**
Erfinder: **Foldi, Gabriele, Dr. Robert-Graf-Strasse 10, A-8010 Graz (AT)**

(74) Vertreter: **Meyer, Hans, 15 rue Viollier, CH-1207 Genève (CH)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 004 522 B1

BUNDESDRUCKEREI BERLIN

# Neue dilignolähnliche Verbindungen, dilignolähnliche Verbindungen zur Verwendung bei der Behandlung von Lebererkrankungen sowie diese enthaltende pharmazeutische Präparate

Die Erfindung betrifft dilignolähnliche Verbindungen zur Verwendung bei der Behandlung für Lebererkrankungen.

Die Erfindung betrifft ferner bestimmte neue Verbindungen, die zur Behandlung von Lebererkrankungen geeignet sind. Als Lignole werden nach K. Freudenberg (Brennstoff-Chemie 44, 328 [1963]; Adv. Chem. Ser. 59,1 [1966]) oligomere Zwischenprodukte der Ligninbildung bezeichnet, die bei der in vivo oder in vitro Dehydrierung von p-Cumaralkohol, Coniferylalkohol oder Sinapinalkohol, die spezielle Verbindungen aus der Klasse der Styrolderivate der allgemeinen Formel

$$Ar-CH=CH-R$$

darstellen ($R=CH_2OH$; Ar=4-Hydroxyphenyl: p-Cumaralkohol, Ar=4-Hydroxy-3-methoxyphenyl: Coniferylalkohol, Ar=4-Hydroxy-3,5-dimethoxyphenyl: Sinapinalkohol), entstehen.

Unter dem Ausdruck »Dilignolähnliche Verbindungen« sind im Zusammenhang mit der vorliegenden Erfindung chemische Substanzen verstanden, die aus Styrolderivaten obiger allgemeiner Formel, worin Ar für einen aromatischen Ring steht, der eine paraständige Hydroxylgruppe als Substituenten trägt und als weitere Substituenten ein oder zwei Methoxygruppen aufweisen kann, wobei die Gesamtzahl der Substituenten maximal drei beträgt, und R eine Methylgruppe ist, durch oxydative Dimerisierung erhalten werden und die nachstehende allgemeine Formel (1) besitzen bzw. durch einfache chemische Transformationen aus den ursprünglichen Dimerisierungsprodukten von Styrolderivaten obiger allgemeiner Formel in die Verbindungen der allgemeinen Formel (1) übergeführt werden können.

In den dilignolähnlichen Verbindungen der allgemeinen Formel (1)

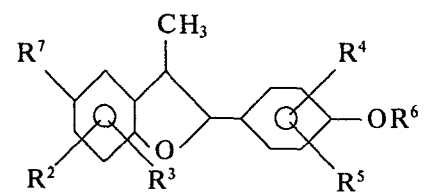

(1)

haben die Symbole erfindungsgemäß die folgenden Bedeutungen:

$R^2$, $R^3$ und $R^4$ sind unabhängig voneinander Wasserstoff oder die Methoxygruppe,

$R^5$ ist Wasserstoff, $-CH_2OH$ oder eine Aminomethylgruppe, die am Stickstoff ein- oder zweifach durch $C_1-C_2$-Alkyl, 2-Hydroxy-äthyl, oder die Carboxymethylgruppe substituiert sein kann,

$R^6$ ist Wasserstoff, eine verzweigte oder unverzweigte $C_1-C_4$-Alkylgruppe, Acetyl, Succinyl, die Hydrogensulfat- oder Dihydrogenphosphatgruppe,

wobei jedoch $R^5$ und $R^6$ nicht gleichzeitig Wasserstoff sein dürfen,

$R^7$ ist die Carboxyl- oder Propylgruppe oder die Gruppe $-CH=CH-CH_3$.

Falls $R^5$ und $R^6$ gleichzeitig Wasserstoff sind, handelt es sich um bekannte Verbindungen (K. V. Sarkanen, Lignins, Occurence, Formation, Structure and Reactions, Wiley, New York, 1971, Seiten 95–164). Diese bekannten Verbindungen weisen, wie auch die neuen Verbindungen, eine Leberschutzwirkungen auf.

Die als Ausgangsmaterial zum Herstellen der neuen Verbindungen dienenden Styrolderivate der eingangs genannten allgemeinen Formel $Ar-CH=CH-CH_3$ sind entweder handelsüblich oder durch einfache chemische Synthesen aus handelsüblichen Stoffen zugänglich.

Die oxidative Dimerisierung der Styrolderivate der angegebenen allgemeinen Formel erfolgt nach bekannten Methoden, wie dies beispielsweise zusammenfassend von H. Musso in dem Buch »Oxidative Coupling of Phenols«, herausgegeben von W. I. Taylor und A. R. Battersby, New York, 1967, auf den Seiten 1 bis 94, oder von K. V. Sarkanen und A. F. A. Wallis im Journal of the Chemical Society, Perkin Transactions I, im Jahre 1973 auf den Seiten 1869 bis 1878 beschrieben ist. Die oxidative Dimerisierung wird vorzugsweise in verdünnter Lösung in den Lösungsmitteln Methanol, Äthanol, 2-Propanol oder Aceton, gegebenenfalls im Gemisch mit Wasser, oder in Pufferlösungen, deren pH-Wert zwischen 4.0 und 8.0 liegt, durchgeführt. Als Oxidationsmittel kommen vor allem Eisen (III)-chlorid, Kaliumhexacyanoferrat (III), Braunstein, Silberoxid, Natriumnitrit, anodische Oxidation oder Wasserstoffperoxid bzw. Sauerstoff in Gegenwart von Enzymen wie Peroxidase oder Laccase in Frage. Die Reaktionstemperatur wird günstigerweise zwischen 0°C und 25°C gewählt. Die Reaktionsdauer liegt in der Regel zwischen 5 und 70 Stunden. Die Aufarbeitung erfolgt durch Abfiltrieren der aus dem Reaktionsansatz ausgefallenen Produkte, gegebenenfalls nach vorherigem Entfernen der organischen Lösungsmittel im Vakuum. Sie führt zu Verbindungen der allgemeinen Formel (1). Die Reindarstellung der Verbindungen erfolgt durch Kristallisation, Verteilungsverfahren oder Chromatographie.

Die auf diese Weise erhaltenen Dimerisierungsprodukte der allgemeinen Formel (1) können nach an sich bekannten Methoden chemischen Weitertransformationen unterworfen werden, was zu weiteren Verbindungen der allgemeinen Formel (1) führt, die für den Verwendungszweck als Wirkstoffe von Lebertherapeutika besonders günstige pharmakologische und galenische Eigenschaften zeigen.

Dazu zählt beispielsweise Verätherung der phenolischen Hydroxylgruppen mit Alkylhalogeniden oder Dialkylsulfaten. Man arbeitet dabei nach an sich üblichen Methoden, wie sie u. a. von H. Meerwein in den »Methoden der Organischen Chemie« (Houben-Weyl), 4. Aufl., herausgegeben von E. Müller, Stuttgart, 1965, im Band 6/3 auf den Seiten 54 bis 67 beschrieben sind. Als Alkylierungsmittel kommen vorzugsweise entsprechende Alkylchloride oder Alkylbromide mit ein bis vier Kohlenstoffatomen in Frage. Die Umsetzung wird in wäßrigem oder alkoholischem Medium durchgeführt. Weitere mögliche Lösungsmittel sind Aceton, 2-Butanon, Cyclopentanon, 1,4-Dioxan, Benzol, Toluol, Xylol oder Chlorbenzol. Die Reaktionen finden in Gegenwart eines Überschusses Base statt, wozu sich insbesondere, je nach Lösungsmittel, die Hydroxyde, Alkoholate oder Carbonate von Lithium, Natrium oder Kalium, aber auch stärkere Basen wie Natriumhydrid eignen. Die Reaktionen können bei Raumtemperatur, aber auch bei erhöhter Temperatur bis zum Siedepunkt des eingesetzten Lösungsmittels durchgeführt werden.

Des weiteren kommt dafür eine Veresterung der phenolischen und Hydroxylgruppen in den Dimerisierungsprodukten der allgemeinen Formel (1) bzw. den daraus durch chemische Weitertransformation erhaltenen Verbindungen in Frage, erzielbar auf an und für sich übliche Art durch Umsetzung mit Säurehalogeniden oder -anhydriden, wie dies beispielsweise von H. Henecka in den »Methoden der Organischen Chemie« (Houben-Weyl), 4. Aufl., herausgegeben von E. Müller, Stuttgart, 1952, im Band 8 auf den Seiten 543 bis 549 beschrieben ist. Zur Darstellung der sauren Bernsteinsäure-ester bewährt sich insbesondere die Veresterung durch Zusammenschmelzen der zu veresternden Verbindung mit Bernsteinsäureanhydrid.

Die Darstellung der sauren Schwefelsäureester bzw. deren Salze erfolgt am günstigsten mittels der Addukte von Schwefeltrioxid an tertiäre Basen wie Triäthylamin, Pyridin oder Dimethylanilin in einem Überschuß einer dieser Basen als Lösungsmittel bei Temperaturen zwischen 0°C und 80°C mit anschließendem Eintragen in wäßrige Lösungen bzw. Suspensionen von Natriumcarbonat, Natriumhydroxid, Kaliumcarbonat oder Bariumhydroxid und Einengen im Vakuum.

Zur Gewinnung der sauren Phosphorsäureester bzw. deren Salze setzt man die entsprechenden Hydroxylverbindungen mit Phosphoroxychlorid in Gegenwart tertiärer Amine, vorzugsweise Pyridin, 2,6-Lutidin, N,N-Dimethylanilin oder Tiräthylamin in Lösungsmitteln wie Äther, Chloroform, Benzol oder Toluol, oder mit einem Überschuß des Amins als Lösungsmittel, bei Temperaturen zwischen −20°C und 80°C um und entfernt sorgfältig alles Flüchtige im Vakuum. Zur Überführung in die entsprechenden Salze versetzt man die auf diese Weise gebildeten Phosphorsäureesterdichloride mit einer wäßrigen Lösung oder Suspension der Hydroxide oder Carbonate der Alkalien oder Erdalkalien, bis ein pH-Wert von 8.5 erreicht wird und engt hierauf entweder im Vakuum ein oder fällt durch Zugabe von Methanol, Äthanol, 2-Propanol oder Aceton. Eine weitere Gruppe chemischer Weitertransformationen an den Dimerisierungsprodukten der allgemeinen Formel (1) umfaßt elektrophile Substitutionen am Phenylkern, wobei nach an sich bekannten Verfahren neue Verbindungen mit wertvollen pharmakologischen Eigenschaften erhalten werden.

Beispielsweise seien in diesem Zusammenhang Hydroxymethylierung mit Formaldehyd in alkalischem Medium, Aminomethylierung mit Formaldehyd und primären oder sekundären Aminen, oder Aminoalkoholen genannt. Der neue Substituent wird bei diesen Verfahren in den Dimerisierungsprodukten der Verbindungen der allgemeinen Formel (1) vorzugsweise orthoständig zu zu einer bereits vorhandenen Hydroxylgruppe eingeführt, sofern diese Kernposition unbesetzt ist.

Die Hydroxymethylierung wird am günstigsten mit wäßriger oder wäßrig-äthanolischer (50/50) Natronlauge oder Kalilauge als Lösungsmittel (Laugenkonzentration 1N bis 6N) und einem zwei- bis zehnfachen Überschuß wäßriger 35%iger Formalinlösung bei Temperaturen zwischen 5°C und 80°C durchgeführt, wobei die Reaktionsdauer zwischen 5 und 24 h beträgt und die Aufarbeitung auf übliche Art durch Ansäuern, Extraktion mit einem geeigneten Lösungsmittel und Umkristallisation erfolgt.

Die Durchführung der Aminomethylierung erfolgt auf an sich übliche Art durch Erwärmen der Dimerisierungsprodukte der allgemeinen Formel (1) mit der äquimolaren bis dreifachen Menge Amin und Aldehyd in Lösungsmitteln wie Methanol, Äthanol oder 2-Propanol bei Temperaturen zwischen 40°C und der Siedetemperatur des Lösungsmittels, wobei die Umsetzungsdauer zwischen 2 und 72 h beträgt. Aus dem Reaktionsansatz ausgefallene Produkte werden abfiltriert, ansonsten engt man im Vakuum zur Trockene ein. Die Reindarstellung erfolgt durch Kristallisation, gegebenenfalls über das Hydrochlorid.

Weitere Möglichkeiten der Weitertransformation ergeben sich beispielsweise für Verbindungen der allgemeinen Formel (1), wenn $R^7$ für die Partialstruktur $-CH=CH-CH_3$ steht, durch katalytische Hydrierung zu $-CH_2-CH_2-CH_3$ oder durch Oxidation, beispielsweise mit Kaliumpermanganat, zu einer Carboxylgruppe.

Zum Gelingen dieser Oxidationsreaktion ist es notwendig, vorher im Substrat allfällig vorhandene freie phenolische Hydroxylgruppen in die entsprechenden Methyläther überzuführen, beispielsweise mit Dimethylsulfat in wäßrig/alkoholischer Lösung. Die Oxidation selbst wird am besten in Aceton

durch portionsweise Zugabe von Kaliumpermanganat durchgeführt, wie dies beispielsweise von H. Erdtmann in Liebigs Annalen der Chemie, Band 503 (1933) auf den Seiten 283 bis 294 beschrieben ist.

Die Erfindung betrifft auch die dilignolähnlichen Verbindungen, sofern sie entsprechende saure oder basische Gruppen besitzen, in Form bestimmter Salze.

Als Salze von Verbindungen mit sauren Gruppen kommen solche mit Lithium, Natrium, Kalium, Calcium oder Magnesium, sowie die Salze mit physiologisch verträglichen Aminen wie N-Methylglucamin, Glucosamin, Dimethylaminoäthanol, Diäthylaminoäthanol, Diäthanolamin, Äthanolamin, Hydroxyäthylpiperazin, Äthylendiamin oder Cholin, oder mit basischen Aminosäuren wie Arginin, Ornithin, Lysin, Carnithin oder Betain in Frage.

Verbindungen mit basischen Gruppen können insbesondere in Form von Salzen mit anorganischen Säuren wie Chlorwasserstoff-, Bromwasserstoff-, Schwefel- oder Phosphorsäure oder mit physiologisch verträglichen organischen Säuren wie Ameisen-, Essig-, Propion-, Bernstein-, Glykol-, Milch-, Apfel-, Wein-, Zitronen-Äthansulfon-, Hydroxyäthansulfon-, Äthylensulfon-, Ascorbin-, Lipon-, Asparagin-, a-Ketoglutar-, Glutamin-, Zucker-, Glucon-, Schleim- oder Thiazolidincarbonsäure eingesetzt werden.

Die wertvollen pharmakologischen Eigenschaften der dilignolähnlichen Verbindungen, der Formel 1 und 1a (siehe Anspruch 1 und 2) können in pharmakologischen Untersuchungen nachgewiesen werden. Standard-Prüfverfahren der Leberschutzwirkung bei experimenteller Leberschädigung sind der Hexobarbital-Schlaftest, der Galactosamintest und der $\alpha$-Amanitintest. Sie sind u. a. beschrieben in der Arbeit von G. Vogel et. al., erschienen in Arzneimittelforschung, Band 25 (1975), auf den Seiten 82 bis 89 und 179 bis 188.

Im Hexobarbital-Schlaftest wurde die Wirkung der dilignolähnlichen Verbindung trans-2,3-Dihydro-2-(4-hydroxy-3-methoxyphenyl)-7-methoxy-3-methyl-5-(E)-propenylbenzofuran (»Dehydrodiisoeugenol«) mit derjenigen des bekannten Lebertherapeutikums Silymarin verglichen. Dazu erhielten Ratten in Gruppen zu je 15 Tieren 100 mg/kg der zu testenden Verbindung in Carboxymethylcellulose intraperitoneal und 90 min später 0.3 ml/kg Tetrachlorkohlenstoff in Olivenöl mittels Schlundsonde. 48 h später wurden die Ratten durch intraperitoneale Verabreichung von 70 mg/kg Hexobarbital narkotisiert, und es wurde die Schlafdauer bestimmt:

| | min |
|---|---|
| Tetrachlorkohlenstoff ohne Testverbindung | 128 |
| Kontrolle (nur Hexobarbital) | 83 |
| trans-2,3-Dihydro-2-(4-hydroxy-3-methoxyphenyl-7-methoxy-3-methyl-5-(E)-propenylbenzofuren (»Dehydrodiisoeugenol«) | 97 |
| Silymarin | 115 |

Die Wirkung von trans-2,3-Dihydro-2-(4-hydroxy-3-methoxyphenyl-7-methoxy-3-methyl-5-(e)-propenylbenzofuran (»Dehydrodiisoeugenol«) ist hochsignifikant ($p<0.01$), jene der Vergleichsverbindung Silymarin statistisch nicht signifikant.

Im Galactosamintest erhielten Ratten in 30 Gruppen zu je 15 Tieren 100 mg/kg der zu testenden neuen Verbindung N-[5-(trans-2,3-Dihydro-7-methoxy-3-methyl-5-(E)-propenylbenzofuran-2-yl)-2-hydroxy-3-methoxyphenylmethyl]-N-(2-hydroxyäthyl)-N-methylammoniumchlorid und zum Vergleich Silymarin in Traganthsuspension peroral verabreicht, eine Stunde später intraperitoneal 350 mg/kg Galactosaminhydrochlorid und 24 h später erfolgte Entnahme der Blutproben zur Bestimmung der Serumenzyme GOT und GPT.

| | GOT | GPT |
|---|---|---|
| Galactosamin-Hydrochlorid ohne Testverbindung | 572 | 578 |
| Kontrolle | 113 | 53 |
| N-[5-(trans-2,3-Dihydro-7-methoxy-3-methyl-5-(E)-propenylbenzofuran-2-yl)-2-hydroxy 3-methoxyphenylmethyl]-N-(2-hydroxyäthyl)-N-methylammoniumchlorid | 422 | 185 |
| Silymarin | 794 | 225 |

Besonders beim Enzym GPT war die Wirkung der getesteten neuen Verbindung hochsignifikant ($p < 0.01$) und deutlich besser als jene von Siylmarin.

Im $\alpha$-Amanitintest erhielten Mäuse in Gruppen zu je 20 Tieren 50 mg/kg N-[5-(trans-2,3-Dihydro-7-methoxy-3-methyl-5-(E)-propenylbenzofuran-2-yl)-2-hydroxy-3-methoxyphenyl-methyl]-N-(2-hydroxyäthyl)-N-methyl-ammoniumchlorid und zum Vergleich Silymarin intravenös verabreicht und eine Stunde später 0,7 mg/kg $\alpha$-Amanitin intraperitoneal. Der Mortalitätsverlauf wurde 7 Tage beobachtet. Silymarin bewirkte keine Verringerung der Mortalitätsfälle gegenüber der Kontrollgruppe, bei N-5-(trans-2,3-Dihydro-7-methoxy-3-methyl-5-(E)-propenyl-benzofuran-2-yl)-2-hydroxy-3-methoxyphenol-methyl]-N-(2-hydroxyäthyl)-N-methylammoniumchlorid betrug die Mortalitätsrate hingegen nur 20%.

Die toxikologischen Untersuchungen zeigten gute Verträglichkeit der vorhin angeführten dilignolähnlichen Verbindungen. So liegt z. B. der $LD_{50}$-Wert bei Ratten nach peroraler Verabreichung für trans-2,3-Dihydro-2-(4-hydroxy-3-methoxyphenyl)-7-methoxy-3-methyl-5-(E)-propenylbenzofuran (»Dehydrodiisoeugenol«) bei über 16 g/kg, jene für N-[5-(trans-2,3-Dihydro-7-methoxy-3-methyl-5-(E)-propenylbenzofuran-2-yl)-2-hydroxy-3-methoxy-phenylmethyl]-N-(2-hydroxyäthyl)-N-methylammoniumchlorid zwischen 4 und 16 g/kg.

Die beanspruchten dilignolähnliche Verbindungen sind daher wertvolle hochaktive Wirkstoffe für Lebertherapeutika.

Die pharmazeutischen Präparate, die die beanspruchten dilignolähnliche Verbindungen als Wirkstoffe enthalten, sind wertvolle Heilmittel gegen Lebererkrankungen und Schutzmittel zur Vorbeugung gegen solche Erkrankungen. Die Dosierung des Wirkstoffes hängt von den Umständen des Einzelfalls, insbesondere der Art der Schädigung und natürlich der Art der Applikation ab, doch liegt die tägliche Dosis in der Regel zwischen 10 und 1000 mg, vor allem zwischen 20 und 500 mg und bevorzugt zwischen 25 und 250 mg aktive Substanz. Die pharmazeutischen Präparate enthalten die dilignolähnlichen Verbindungen in freier Form oder in Form der therapeutisch verwendbaren Salze, in der Regel in Mischung mit einem für die enterale oder parenterale, insbesondere orale, rektale oder intravenöse Applikation geeigneten pharmazeutischen anorganischen oder organischen Trägermaterial. Als solches kommen Stoffe in Betracht, die mit den Wirkstoffen nicht reagieren, z. B. Wasser, Gelatine, Lactose, Stärke, Stearylalkohol, Magnesiumstearat, Talk, pflanzliche Öle, Benzylalkohole, Gummi, Propylenglykole, Vaseline und andere bekannte Arzneimittelträger. Die pharmazeutischen Präparate können z. B. als Tabletten, Dragées, Kapseln, z. B. Gelatinekapseln, Suppositorien, oder in flüssiger Form als Lösungen (z. B. Elixier oder Sirup), Suspensionen oder Emulsionen vorliegen. Gegebenenfalls sind sie sterilisiert und/oder enthalten sie Hilfsstoffe, wie Konservierungs-, Stabilisierungs-, Netz- oder Emulgiermittel, Lösungsvermittler oder Salze zur Veränderung des osmotischen Drucks oder Puffer. Sie können auch andere therapeutische wertvolle Substanzen enthalten. Die pharmazeutischen Präparate werden nach üblichen Methoden gewonnen.

Die nun folgenden Beispiele dienen der Illustration der Synthese neuer dilignolähnlicher Verbindungen und zur Herstellung der pharmazeutischen Präparate.

## Beispiel 1

Man hält 80 g Phosphoroxychlorid, 14,5 g N,N-Dimethylanilin und 30 g trans-2,3-Dihydro-2-(4-hydroxy-3-methoxyphenyl)-7-methoxy-3-methyl-5-(E)-propenyl-benzofuran (»Dehydrodiisoeugenol«) in 400 ml absolutem Toluol 12 h bei Raumtemperatur und hierauf 3 h bei 70° C, trennt nach dem Abkühlen vom ausgeschiedenen Hydrochlorid des N,N-Dimethylanilins ab und entfernt alles Flüchtige sorgfältig in Vakuum bei 60° C. Das verbleibende zähe Öl wird mit 100 ml destilliertem Wasser versetzt und bei 60° C unter ständigem Umschwenken portionsweise festes Natriumcarbonat zugegeben, bis keine Kohlendioxidentwicklung mehr zu beobachten ist. Man läßt auf Raumtemperatur abkühlen und stellt durch Zugabe von 2 N Natronlauge den pH-Wert der Lösung auf 8,5. Hierauf versetzt man unter heftigem Rühren mit 2 l 96%ige Äthanol, wobei sich ein weißer flockiger Niederschlag bildet, der sich sofort von der Mutterlauge abgetrennt und im Vakuum über $P_4O_{10}$ getrocknet wird. Nach Wiederholung dieser Umfällung werden 33 g Dinatrium-4-[trans-2,3-dihydro-7-methoxy-3-methyl-5-(E)-propenylbenzofuran-2-yl]-2-methoxyphenylphosphat erhalten; feine hygroskopische Kristalle, Fp. 185 – 187° (Zers.).

$C_{20}H_{21}O_7PNa_2$ (450,16)

| | | | | |
|---|---|---|---|---|
| Ber. | C 53,34 | H 4,70 | P 6,87 | Na 10,21 |
| Gef. | C 52,91 | H 4,92 | P 6,43 | Na 10,02 |

### Herstellung des Ausgangsmaterials

Zu einer Lösung von 100 g (E)-Isoeugenol in 1,3 l 75%ige Äthanol gibt man 150 g Eisen-(III)-chlorid in 400 ml Wasser und läßt 24 h bei 0° stehen. Der gebildete Niederschlag wird abfiltriert und aus Äthanol

umkristallisiert, wobei 53 g trans-2,3-Dihydro-2-(4-hydroxy-3-methoxyphenyl-7-methoxy-3-methyl-5-(E)-propenylbenzofuran (»Dehydrodiisoeugenol«) als farblose Kristalle erhalten werden; Fp. 132–133°.

## Beispiel 2

Man hält 5 g trans-2,3-Dihydro-2-(4-hydroxy-3-methoxyphenyl-7-methoxy-3-methyl-5-(E)-propenylbenzofuran (»Dehydrodiisoeugenol«) und 1,36 g N-Methyl-glycin in 50 ml Äthanol nach Versetzen mit 1,22 ml 35 proz. Formalinlösung 16 h unter Rückfluß, wobei nach 12 h nochmals die gleiche Menge N-Methylglycin und Formalinlösung zugesetzt wird. Nach Abkühlen trennt man den gebildeten Niederschlag ab und hält nach Umkristallisation aus Methanol/Wasser 4,1 g N-[5-(trans-2,3-Dihydro-7-methoxy-3-methyl-5-(E)-propenylbenzofuran-2-yl(-2-hydroxy-3-methoxyphenylmethyl]-N-methylglycin; feine weiße Kristalle, Fp. 140,5–141,5° (Zers.).

$C_{24}H_{29}O_6N$ (427,29)

| | | | |
|---|---|---|---|
| Ber. | C 67,40 | H 6,86 | N 3,28 |
| Gef. | C 67,13 | H 6,73 | N 3,21 |

## Beispiel 3

Man hält 5 g trans-2,3-Dihydro-2-(4-hydroxy-3-methoxyphenyl-7-methoxy-3-methyl-5-(E)-propenylbenzofuran (»Dehydrodiisoeugenol«), 0,9 g Paraformaldehyd und 2,3 g N-Methylaminoäthanol in 60 ml absol. Äthanol 48 h bei 65°, entfernt hierauf das Lösungsmittel i. Vak. und erhält nach Kristallisation des Rückstands aus Methylenchlorid/Petroläther (Kp. 60–80°) 4 g N-[5-(trans-2,3-Dihydro-7-methoxy-3-methyl-5-(E)-propenylbenzofuran-2-yl)-2-hydroxy-3-methoxyphenylmethyl]-N-(2-hydroxyäthyl)-N-methylamin; feine farblose Kristalle, Fp. 101–103° C.

$C_{24}H_{31}O_5N$ (413,31)

| | | | |
|---|---|---|---|
| Ber. | C 69,68 | H 7,58 | N 3,39 |
| Gef. | C 69,59 | H 7,51 | N 3,17 |

## Beispiel 4

Zur Herstellung des Hydrochlorids der nach Beispiel 3 erhaltenen Verbindung tropft man zu einer Lösung von 5 g N-[5-(trans-2,3-Dihydro-7-methoxy-3-methyl-5-(E)-propenylbenzofuran-2-yl)-2-hydroxy-3-methoxyphenylmethyl]-N-(2-hydroxyäthyl)-N-methylamin in 60 ml absol. Äther die äquimolare Menge ätherische Salzsäure. Der ausgefallene Niederschlag wird abgesaugt und mit absol. Äther gewaschen. Man erhält 8,8 g N-[5-(trans-2,3-Dihydro-7-methoxy-3-methyl-5-(E)-propenylbenzofuran-2-yl)-2-hydroxy-3-methoxyphenylmethyl]-N-(2-hydroxyäthyl)-N-methylammoniumchlorid; feine schwach gelbliche hygroskopische Kristalle, Fp. 74° (Zers.).

$C_{24}H_{32}ClNO_5$ (449,77)

| | | | | |
|---|---|---|---|---|
| Ber. | C 64,03 | H 7,19 | Cl 7,88 | N 3,11 |
| Gef. | C 63,79 | H 7,13 | Cl 7,77 | N 3,02 |

Zur Herstellung des Aspartats gibt man zu einer Lösung von 1 g N-[5-(trans-2,3-Dihydro-7-methoxy-3-methyl-5-(E)-propenylbenzofuran-2-yl)-2-hydroxy-3-methoxy-phenylmethyl]-N-(2-hydroxyäthyl)-N-methylamin in 25 ml Äthanol 0,32 g L-Asparginsäure in 15 ml heißem Wasser. Man hält 2 h auf 70° und zieht anschließend das Lösungsmittel ab. Nach Trocknen im Vakuum wird ein schwach bräunlicher amorpher Rückstand erhalten; N-[5-(trans-2,3-Dihydro-7-methoxy-3-methyl-5-(E)-propenylbenzofuran-2-yl)-2-hydroxy-3-methoxyphenylmethyl]-N-(2-hydroxyäthyl)-N-methylammoniumasparat.

Zur Herstellung des Salzes mit Liponsäure fügt man zu einer Lösung von 1 g N-[5-(trans-2,3-Dihydro-7-methoxy-3-methyl-5-(E)-propenylbenzofuran-2-yl)-2-hydroxy-3-methoxyphenylmethyl]-N-(2-hydroxyäthyl)-N-methylamin in 50 ml Methanol 0.5 g Liponsäure zu, rührt 2 h bis zur vollständigen Homogenisierung und erhält nach Verdampfen des Methanols und Trocknen i. Vak. einen hellbraunen amorphen Rückstand.

## Beispiel 5

Man löst 3.9 g Ornithin in 200 ml Wasser und versetzt mit einer Lösung von 10 g trans-2,3-Dihydro-2-(3,4-dimethoxyphenyl)-7-methoxy-3-methylbenzofuran-5-carbonsäure in 2 lÄtha-

nol. Die zunächst klare Lösung beginnt sich nach etwa 30 min zu trüben und es beginnt Ausfällung eines voluminösen Niederschlags, der nach 2 h abgesaugt, mit 95 proz. Äthanol gewaschen und getrocknet wird. Nach Umkristallisation aus Wasser/Aceton erhält man 10 g farblose Kristalle von L-Ornithin trans-2,3-Dihydro-2-(3,4-dimethoxyphenyl) 7-methoxy-3-methylbenzofuran-5-carboxylat, Fp. 192—193° (Zers.).

$C_{24}H_{32}N_2O_8$ (476,32)

| | | | |
|---|---|---|---|
| Ber. | C 60,46 | H 6,79 | N 5,88 |
| Gef. | C 60,19 | H 6,66 | N 5,79 |

Herstellung des Ausgangsmaterials

Man versetzt 40 g trans-2,3-Dihydro-2-(4-hydroxy-3-methoxyphenyl)-methoxy-3-methyl-5-(E)-propenyl-benzofuran in 800 ml 95proz. Äthanol mit 30 ml Dimethylsulfat und hierauf portionsweise unter Schütteln mit insgesamt 48 ml 30proz. Kalilauge, wobei die Temperatur von 40° nicht überschritten werden soll. Anschließend rührt man noch 12 h bei Raumtemperatur und verdünnt hierauf mit 1 l Wasser. Der gebildete Niederschlag wird abgesaugt und mit Wasser gewaschen. Nach Umkristallisation aus Äthanol werden 34.8 g trans-2,3-Dihydro-2-(3,4-dimethoxyphenyl)-7-methoxy-3-methyl-5-(E)-propenylbenzofuran erhalten; Fp. 124°.

Zu 15 g dieses methylierten Produkts in 300 ml absol. Aceton gibt man unter Rühren und Eiskühlung innerhalb von 16 h in kleinen Portionen insgesamt 21 g Kaliumpermanganat, verdampft das Aceton i. Vak., suspendiert den Rückstand in 600 ml Wasser und leitet Schwefeldioxid bis zum Gelbwerden der Lösung ein, wobei der Braunstein in Lösung geht und ein neuer flockiger Niederschlag ausfällt. Dieser wird abgetrennt, mit Wasser gewaschen, mit 600 ml 10proz. Natronlauge digeriert, vom Ungelösten abzentrifugiert und der Rückstand viermal erneut mit je 200 ml Wasser digeriert. Die vereinigten wäßrig-alkalischen Phasen säuert man mit verd. Salzsäure an und erhält nach Umkristallisation, zuerst aus Essigester/Petroläther (Kp. 60—80°), anschließend aus Eisessig 6.4 g trans-2,3-Dihydro-2-(3,4-dimethoxyphenyl)-7-methoxy-3-methylbenzofuran-5-carbonsäure, Fp. 135—136°.

Zur Freisetzung von L-Ornithin gibt man das entsprechende Hydrochlorid auf eine Austauschersäule mit Amberlite 120 in der $NH_4^+$-Form, eluiert anschließend mit 5proz. Ammoniak und verdampft alles Flüchtige i. Vak.

Zur Herstellung des Salzes mit L-Arginin versetzt man eine Lösung von 5.1 g L-Arginin in 200 ml Wasser mit 10 g trans-2,3-Dihydro-2-(3,4-dimethoxy-phenyl)-7-methoxy-3-methylbenzofuran-5-carbonsäure in 500 ml 95proz. Äthanol. Man erwärmt 2 h auf 60° und entfernt anschließend die Lösungsmittel i. Vak., wobei ein schwach gelblicher kristalliner Rückstand hinterbleibt: L-Arginin trans-2,3-Dihydro-2-(3,4-dimethoxyphenyl)-7-methoxy-3-methylbenzofuran-5-carboxylatmonohydrat, Fp. 130—135°.

$C_{25}.H_{34}.N_4O_8.H_2O$ (536,40)

| | | |
|---|---|---|
| Ber. | C 55,93 | H 6,78 |
| Gef. | C 55,56 | H 6,82 |

Zur Herstellung des Salzes mit Cholin versetzt man 4.1 g Cholinchlorid in 150 ml absol. Äthanol unter Rühren mit 1.6 g KOH in 6 ml Wasser, filtriert nach 2 h vom ausgefallenen KCl ab und wäscht dieses mit absol. Äthanol nach. Zu dieser Lösung fügt man tropfenweise 10 g trans-2,3-Dihydro-2-(3,4-dimethoxyphenyl)-7-methoxy-3-methylbenzofuran-5-carbonsäure in 500 ml absol. Äthanol zu und hält anschließend 1 h auf 65°.

Anschließend wird i. Vak. eingeengt und durch Zugabe von Diäthyläther 13 g Cholin trans-2,3-Dihydro-2-(3,4-dimethoxyphenyl)-7-methoxy-3-methylbenzofuran-5-carboxylat-monohydrat erhalten; Fp. 163—165°.

$C_{24}H_{33}NO_5.H_2O$ (465,38)

| | | |
|---|---|---|
| Ber. | C 61,89 | H 7,59 |
| Gef. | C 61,84 | H 7,48 |

Beispiel 6

Man löst unter Stickstoff 10 g trans-2,3-Dihydro-2-(4-hydroxy-3-methoxyphenyl)-7-methoxy-3-methyl-5-(E)-propenylbenzofuran in 400 ml 2 N Kalilauge und versetzt mit 37 ml 35proz. Formalinlösung. Hierauf hält man 24 h auf 75° und versetzt innerhalb dieser Zeit noch zweimal mit der gleichen Menge Formalinlösung. Nach dem Abkühlen säuert man mit verd. Salzsäure bis zu pH 4 an, extrahiert mit Äther, wäscht die Ätherphase mit Wasser, 5proz. $NaHCO_3$-Lösung und Wasser und trocknet über

$Na_2SO_4$. Das nach Entfernen des Äthers zurückbleibende Öl wird aus Methylenchlorid/Petroläther (Kp. 60—80°) kristallisiert und gibt 7 g 5-[trans-2,3-Dihydro-7-methoxy-3-methyl-5-(E)-propenylbenzofuran-2-yl]-2-hydroxy-3-methoxybenzylalkohol, Fp. 117—119°.

$C_{21}H_{24}O_5$ (356,24)

| | | |
|---|---|---|
| Ber. | C 70,74 | H 6,80 |
| Gef. | C 70,93 | H 6,67 |

Beispiel 7

Herstellung pharmazeutischer Präparate

a) Herstellung von 10 000 Tabletten mit einem Gehalt von je 50 mg der aktiven Substanz

Bestandteile:

| | |
|---|---|
| trans-2,3-Dihydro-2-(4-hydroxy-3-methoxyphenyl)-7-methoxy-3-methyl-5-(E)-propenylbenzofuran | 500 g |
| Milchzucker | 1700 g |
| Maisstärke | 90 g |
| Polyäthylenglykol 6000 | 90 g |
| Talkpulver | 90 g |
| Magnesiumstearat | 30 g |

Verfahren

Die pulvrigen Bestandteile werden mit einem Sieb von 0.6 mm Maschenweite gesiebt. Dann wird der Wirkstoff mit Milchzucker, Talk, Magnesiumstearat und der Hälfte der Stärke in einem geeigneten Mischer vermischt. Die andere Hälfte der Stärke wird in 50 ml Wasser suspendiert und die Suspension zu einer 80° heißen Lösung des Polyäthylenglykols in 190 ml Wasser gegeben. Die auf diese Weise erhaltene Paste wird zu der Mischung der pulvrigen Bestandteile gegeben, und man granuliert, gegebenenfalls unter Zugabe einer weiteren Wassermenge. Das Granulat wird 12 h bei 30° getrocknet, durch ein Sieb von 1.2 mm Maschenweite getrieben und zu Tabletten mit 7 mm Durchmesser verpreßt.

b) Herstellung von 10 000 Kapseln mit einem Gehalt von je 100 mg der aktiven Substanz

Bestandteile:

| | |
|---|---|
| N-[5-(trans-2,3-Dihydro-7-methoxy-3-methyl-5-(E)-propenylbenzofuran-2-yl)-2-hydroxy-3-methoxy-phenylmethyl]-N-(2-hydroxyäthyl)-N-methylamin | 1000 g |
| Milchzucker | 2800 g |
| Talkpulver | 200 g |

Verfahren

Die pulvrigen Bestandteile werden mit einem Sieb von 0.6 mm Maschenweite gesiebt. Dann wird der Wirkstoff in einem Mischer zuerst mit dem Talkpulver und dann mit dem Milchzucker homogenisiert. Unter Verwendung einer Füllmaschine werden Gelatinekapseln entsprechender Größe mit je 400 mg Gemisch gefüllt.

c) Herstellung von 10 000 ml Injektionslösung zur Abfüllung in Ampullen

Bestandteile:

| | |
|---|---|
| N-[5-(trans-2,3-Dihydro-7-methoxy-3-methyl-5-(E)-propenylbenzofuran-2-yl)-2-hydroxy-)-3-methoxyphenylmethyl]-N-(2-hydroxyäthyl-N-methylammoniumchlorid | 600 g |
| Natriumchlorid | 10 g |
| Aqua pro injectione | ad 10 000 ml |

Verfahren

Nach dem Auflösen der Bestandteile filtriert man durch eine Glasfilternutsche der Durchlässigkeit G 3 und füllt hierauf unter Stickstoff in Ampullen ab, zur Verwendung für i. v.-Injektionen in solche von 2 ml Inhalt, bei Verwendung als Zusatz von Infusionen in solche zu 5 ml Inhalt.

**Patentansprüche**

1. Dilignolähnliche Verbindungen der Formel (1)

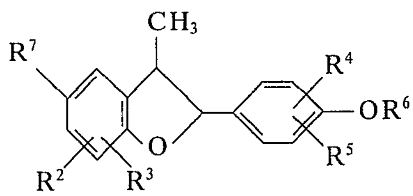

(1)

worin die Symbole die folgenden Bedeutungen haben:
$R^2$, $R^3$ und $R^4$ sind unabhängig voneinander Wasserstoff oder die Methoxygruppe,
$R^5$ ist Wasserstoff, $-CH_2OH$ oder eine Aminomethylgruppe, die am Stickstoff ein- oder zweifach durch $C_1-C_2$-Alkyl, 2-Hydroxy-äthyl oder die Carboxymethylgruppe substituiert sein kann,
$R^6$ ist Wasserstoff, eine verzweigte oder unverzweigte $C_1-C_4$-Alkylgruppe, Acetyl, Succinyl, die Hydrogensulfat- oder Dihydrogenphosphatgruppe,
wobei jedoch $R^5$ und $R^6$ nicht gleichzeitig Wasserstoff sein dürfen,
$R^7$ ist die Carboxyl- oder Propylgruppe oder die Gruppe $-CH=CH-CH_3$-
sowie Salze dieser Verbindungen mit a) Lithium, Natrium, Kalium, Magnesium, Calcium oder physiologisch verträglichen Aminen, sofern saure Gruppen vorliegen, oder mit b) physiologisch verträglichen Säuren, sofern basische Gruppen vorliegen.

2. Verbindungen der Formel (1a) zur Verwendung bei der Behandlung von Lebererkrankungen

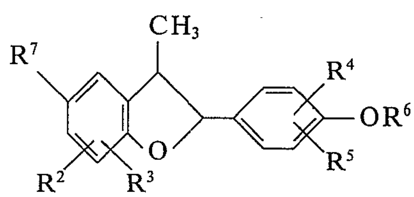

(1a)

worin die Symbole die folgenden Bedeutungen haben:
$R^2$, $R^3$ und $R^4$ sind unabhängig voneinander Wasserstoff oder die Methoxygruppe,
$R^5$ ist Wasserstoff, $-CH_2OH$ oder eine Aminomethylgruppe, die am Stickstoff ein- oder zweifach durch $C_1-C_2$-Alkyl, 2-Hydroxyäthyl oder die Carboxymethylgruppe substituiert sein kann,
$R^6$ ist Wasserstoff, eine verzweigte oder unverzweigte $C_1-C_4$-Alkylgruppe, Acetyl, Succinyl, die Hydrogensulfat- oder Dihydrogenphosphatgruppe,
$R^7$ ist die Carboxyl- oder Propylgruppe oder die Gruppe $-CH=CH-CH_3$,
sowie von Salzen dieser Verbindungen mit a) Lithium, Natrium, Kalium, Magnesium, Calcium oder physiologisch verträglichen Aminen, sofern saure Gruppen vorliegen, oder mit b) physiologisch verträglichen Säuren, sofern basiche Gruppen vorliegen.

3. L-Ornithin trans 2,3-Dihydro-2-(3,4-dimethoxyphenyl)-7-methoxy-3-methylbenzofuran-5-carboxylat, L-Arginin trans-2,3-Dihydro-2-(3,4-dimethoxyphenyl)-7-methoxy-3-methylbenzofuran-5-carboxylat-monohydrat, Cholin trans-2,3-Dihydro-2-(3,4-dimethoxyphenyl)-7-methoxy-3-methylbenzofuran-5-carboxylat-monohydrat.

4. Dinatrium-4-[trans-2,3-dihydro-7-methoxy-3-methyl-5-(E)-propenylbenzofuran-2-yl]-2-methoxyphenylphosphat.

5. N-[5-(trans-2,3-Dihydro-7-methoxy-3-methyl-5-(E)-propenyl-benzofuran-2-yl)-2-hydroxy-3-methoxy-phenylmethyl]-N-methylglycin.

6. N-[5-(trans-2,3-Dihydro-7-methoxy-3-methyl-5-(E)-propenyl-benzofuran-2-yl)-2-hydroxy-3-methoxy-phenylmethyl]-N-(2-hydroxyäthyl)-N-methylamin, sowie dessen Hydrochlorid, L-Aspartat und alpha-Liponat.

7. 5-[trans-2,3-Dihydro-7-methoxy-3-methyl-5-(E)-propenylbenzofuran-2-yl]-2-hydroxy-3-methoxybenzylalkohol.

8. 2,3-Dihydro-2-(4-hydroxy-3-methoxyphenyl-7-methoxy-3-methyl-5-propenylbenzofuran (»Dehydrodiisoeugenol«) nach Anspruch 2, zur Verwendung bei der Behandlung von Lebererkrankungen.

9. 2,3-Dihydro-3-(4-hydroxy-3-methoxyphenyl)-7-methoxy-3-methyl-5-propylbenzofuran (»Dihydrodehydrodiisoeugenol«) nach Anspruch 2, zur Verwendung bei der Behandlung von Lebererkrankungen.

10. 2,3-Dihydro-2-(3,4-dimethoxyphenyl-7-methoxy-3-methyl-benzofuran-5-carbonsäure nach Anspruch 2, zur Verwendung bei der Behandlung von Lebererkrankungen.

11. Pharmazeutisches Präparat gegen Lebererkrankungen, gekennzeichnet durch einen Gehalt einer pharmakologisch wirksamen Menge mindestens einer Verbindung nach einem der Ansprüche 1—10.

## Claims

1. Dilignol type compounds of formula (1)

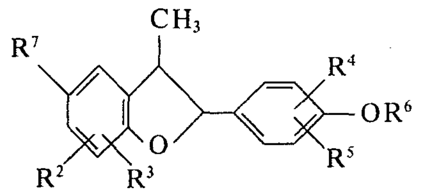

(1)

wherein the symbols have the following significations:
$R^2$, $R^3$ and $R^4$ are independently from each other hydrogen or the methoxy group,
$R^5$ is hydrogen, $-CH_2OH$ or an aminomethyl group, which can be mono- or disubstituted on the nitrogen by $C_1-C_2$-alkyl, 2-hydroxyethyl or the carboxymethyl group,
$R^6$ is hydrogen, a branched or unbranched $C_1-C_4$-alkyl group, acetyl, succinyl, the hydrogensulfate or dihydrogenphosphate group,
with the proviso that not both $R^5$ and $R^6$ are hydrogen at the same time,
$R^7$ is the carboxyl or propyl group or the group $-CH=CH-CH_3$,
as well as salts of these compounds with a) lithium, sodium, potassium, magnesium, calcium or physiologically compatible amines, if acid groups are present, or with b) physiologically acceptable acids, if basic groups are present.

2. Compounds of formula (1a) for use in the treatement of liver diseases

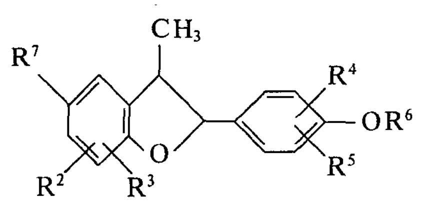

(1a)

wherein the symbols have the following significations:
$R^2$, $R^3$ and $R^4$ are independently from each other hydrogen or the methoxy group,
$R^5$ is hydrogen, $-CH_2OH$ or an aminomethyl group, which can be mono- or disubstituted on the nitrogen by $C_1-C_2$-alkyl, 2-hydroxyethyl or the carboxymethyl group,
$R^6$ is hydrogen, a branched or unbranched $C_1-C_4$-alkyl group, acetyl, succinyl, the hydrogensulfate or dihydrogenphosphate group,
$R^7$ is the carboxyl or propyl group or the group $-CH=CH-CH_3$,
as well as salts of these compounds with a) lithium, sodium, potassium, magnesium, calcium or physiologically compatible amines, if acid groups are present, or with b) physiologically acceptable acids, if basic groups are present.

3. L-Ornithine trans-2,3-dihydro-2-(3,4-dimethoxyphenyl)-7-methoxy-3-methylbenzofuran-5-carboxylate, L-arginine trans-2,3-dihydro-2-(3,4-dimethoxyphenyl)-7-methoxy-3-methylbenzofuran-5-carboxylate-monohydrate, choline trans-2,3-dihydro-2-(3,4-dimethoxyphenyl)-7-methoxy-3-methylbenzofuran-5-carboxylate-monohydrate.

4. Disodium-4-[trans-2,3-dihydro-7-methoxy-3-methyl-5-(E)-propenylbenzofuran-2-yl]-2-methoxyphenylphosphate.

5. N-[5-(trans-2,3-Dihydro-7-methoxy-3-methyl-5-(E)-propenyl-benzofuran-2-yl)-2-hydroxy-3-methoxy-phenylmethyl]-N-methylglycine.

6. N-[5-(trans-2,3-Dihydro-7-methoxy-3-methyl-5-(E)-propenyl-benzofuran-2-yl)-2-hydroxy-3-methoxy-phenylmethyl]-N-(2-hydroxyäthyl)-N-methylamine, and its chlorohydrate, L-aspartate and alpha-liponate.

7. 5-[trans-2,3-Dihydro-7-methoxy-3-methyl-5-(E)-propenylbenzofuran-2-yl]-2-hydroxy-3-methoxybenzylalcohol.

8. 2,3-Dihydro-2-(4-hydroxy-3-methoxyphenyl-7-methoxy-3-methyl-5-propenylbenzofuran (»Dehydrodiisoeugenol«) according to claim 2, for use in the treatment of liver diseases.

9. 2,3-Dihydro-3-(4-hydroxy-3-methoxyphenyl)-7-methoxy-3-methyl-5-propylbenzofuran (Dihydrodehydrodiisoeugenol) according to claim 2, for use in the treatment of liver diseases.

10. 2,3-Dihydro-2-(3,4-dimethoxyphenyl-7-methoxy-3-methyl-benzofuran-5-carboxylate according to claim 2, for use in the treatment of liver diseases.

11. Pharmaceutical preparations against liver diseases, characterized by a pharmacological active amount of at least one compound according to one of claims 1 to 10.

**Revendications**

1. Composés du type dilignol, de formule (1)

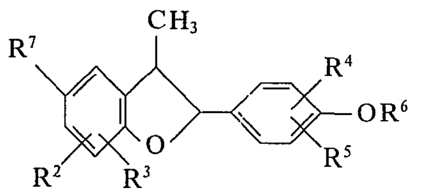

(1)

dans laquelle les symboles ont les significations suivantes:
$R^2$, $R^3$ et $R^4$ sont indépendamment l'un de l'autre de l'hydrogène ou le groupe méthoxy,
$R^5$ est de l'hydrogène, $-CH_2OH$ ou un groupe aminométhyle, qui peut être substitué à l'azote une ou deux fois par $C_1-C_2$-alkyle, 2-hydroxyéthyle ou le groupe carboxyméthyle,
$R^6$ est de l'hydrogène, un groupe $C_1-C_4$-alkyle branché ou non branché, acétyle, succinyle, le groupe hydrogène-sulfate ou dihydrogène-phosphate,
à condition toutefois que $R^5$ et $R^6$ ne peuvent pas être de l'hydrogène en même temps,
$R^7$ est le groupe carboxyle, propyle ou $-CH=CH-CH_3$,
ainsi que les sels de ces composés avec a) le lithium, sodium, potassium, magnésium, calcium ou une amine physiologiquement compatible, si des groupes acides sont présent, ou avec b) des acides physiologiquement compatibles, si des groupes basiques sont présents.

2. Composés de formule (1a) utilisables pour le traitement de maladies du foie

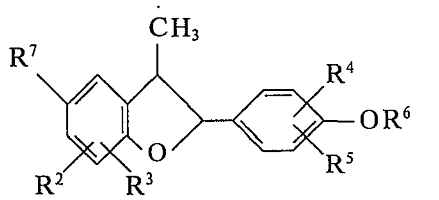

(1a)

dans laquelle les symboles ont les significations suivantes:
$R^2$, $R^3$ et $R^4$ sont indépendamment l'un de l'autre de l'hydrogène ou le groupe méthoxy,
$R^5$ est de l'hydrogène, $-CH_2OH$ ou un groupe aminométhyle, qui peut être substitué à l'azote une ou deux fois par $C_1-C_2$-alkyle, 2-hydroxyéthyle ou le groupe carboxyméthyle,
$R^6$ est de l'hydrogène, un groupe $C_1-C_4$-alkyle branché ou non branché, acétyle, succinyle, le groupe hydrogènesulfate ou dihydrogènephosphate,
$R^7$ est le groupe carboxyle, propyle ou $-CH=CH-CH_3$,
ainsi que les sels de ces composés avec a) le lithium, sodium, potassium, magnésium, calcium ou une amine physiologiquement compatible, si des groupes acides sont présents, ou avec b) des acides physiologiquement compatibles, si des groupes basiques sont présents.

3. L-Ornithine trans-2,3-dihydro-2-(3,4-diméthoxyphényl)-7-méthoxy-3-méthylbenzofuranne-5-carboxylate, L-arginine trans-2,3-dihydro-2-(3,4-diméthoxyphényle)-7-méthoxy-3-méthylbenzofuranne-5-carboxylate-monohydrate, choline trans-2,3-dihydro-2-(3,4-diméthoxyphényle)-7-méthoxy-3-méthylbenzofuranne-5-carboxylate-monohydrate.

4. Disodium-4-[trans-2,3-dihydro-7-méthoxy-3-méthyl-5-(E)-propénylbenzofuran-2-yl]-2-méthoxyphénylphosphate.

5. N-[5-(trans-2,3-dihydro-7-méthoxy-3-méthyl-5-(E)-propényl-benzofuran-2-yl)-2-hydroxy-3-méthoxy-phénylméthyl]-N-méthylglycine.

6. N-[5-(trans-2,3-dihydro-7-méthoxy-3-méthyl-5-(E)-propényl-benzofuran-2-yl)-2-hydroxy-3-méthoxy-phénylméthyl]-N-(2-hydroyéthyl)-N-méthylamine, ainsi que son chlorehydrate, L-aspartate et alpha-liponate.

7. 5-[trans-2,3-dihydro-7-méthoxy-3-méthyl-5-(E)-propénylbenzofuran-2-yl]-2-hydroxy-3-méthoxybenzylol.

8. 2,3-Dihydro-2-(4-hydroxy-3-méthoxyphényl)-7-méthoxy-3-méthyl-5-propénylbenzofuranne (»déhydrodiisoeugénol«) selon la revendication 2, pour l'utilisation pour le traitement de maladies du foie.

9. 2,3-Dihydro-3-(4-hydroxy-3-méthoxyphényl)-7-méthoxy-3-méthyl-5-propénylbenzofuranne (»dihydrodéhydrodiisoeugénol«) selon la revendication 2, pour l'utilisation pour le traitement de maladies du foie.

10. 2,3-Dihydro-2-(3,4-diméthoxyphényl)-7-méthoxy-3-méthylbenzofuran-5-carboxylate, selon la revendication 2, pour l'utilisation pour le traitement de maladies du foie.

11. Préparation pharmaceutique contre des maladies du foie, caractérisée par une quantité pharmacologiquement active d'au moins un composé selon une des revendications 1 à 10.